# EUROPEAN PATENT SPECIFICATION

(11) **EP 4 631 467 B1**
(45) Date of publication and mention of the grant of the patent: **04.11.2026**
(21) Application number: 25181760.7
(22) Date of filing: 25.09.2020
(51) Int. Cl.: A61B 17/34, A61B 90/11

(54) **FRONT-LOADABLE FLUID TRANSFER ASSEMBLIES AND RELATED MEDICAL FLUID TRANSFER SYSTEMS**
VON VORNE BELADBARE FLÜSSIGKEITSÜBERTRAGUNGSANORDNUNGEN UND ZUGEHÖRIGE MEDIZINISCHE FLÜSSIGKEITSÜBERTRAGUNGSSYSTEME
ENSEMBLES DE TRANSFERT DE FLUIDE CHARGEABLES À L'AVANT ET SYSTÈMES DE TRANSFERT DE FLUIDE MÉDICAL ASSOCIÉS

(30) Priority: 19.12.2019 US 201962950521 P; 09.09.2020 US 202063076008 P
(43) Date of publication of application: 15.10.2025
(62) Document number(s) of the earlier application(s) in accordance with Art. 76 EPC: 20792792.2 / 4 041 105
(73) Proprietor: ClearPoint Neuro, Inc., Solana Beach, CA 92075 (US)
(72) Inventor: DALY, Maxwell Jerad, Redlands, CA 92373 (US); PIFERI, Peter G., Orange, CA 92867 (US)
(74) Representative: Yeadon IP Limited

(56) References cited:
- EP-A2- 2 484 402
- EP-A2- 2 558 154
- US-A- 4 955 863
- US-A1- 2007 196 414
- US-A1- 2017 151 416
- US-A1- 2017 232 229
- US-B2- 9 891 296

## Description

### RELATED APPLICATIONS

This application claims the benefit of and priority to U.S. Provisional Patent Application Serial Number 62/950,521 filed December 19, 2019, and U.S. Provisional Patent Application Serial Number 63/076,008 filed September 9, 2020.

### FIELD OF THE INVENTION

The present invention relates generally to medical devices and systems and, more particularly, to devices and systems for delivering and/or withdrawing substances *in vivo.*

### BACKGROUND

Various therapeutic and diagnostic procedures require that a substance be delivered (e.g., infused) into or aspirated from a prescribed region of a patient, such as to an intrabody target using a delivery device. It may be important or critical that the substance be delivered or removed with accuracy to the target region in the patient and without undue trauma to the patient. US4955863 A, US2017/232229 A1, US9891296 B2, EP2558154 A2, US2017/151416 A1, EP2484402 A2 and US2007/196414 A1 disclose devices of the prior art.

### SUMMARY

1 inch = 25,4 mm, 1 foot = 304. 8 mm.

Claim 1 defines the invention and dependent claims disclose embodiments. No surgical methods are claimed per se. It should be appreciated that this Summary is provided to introduce a selection of concepts in a simplified form, the concepts being further described below in the Detailed Description. This Summary is not intended to identify key features or essential features of this disclosure, nor is it intended to limit the scope of the invention.

Embodiments of the invention are directed to front-loadable fluid transfer assemblies for transferring fluid to or from a subject.

Embodiments of the disclosure are directed to a surgical plunger assembly that includes: a stylet comprising opposing proximal and distal ends; a luer connector comprising an internal seal coupled to the stylet; and a support body coupled to the luer connector and extending above the distal end of the stylet. The support body encloses a sub-length of the stylet and the stylet is longitudinally moveable relative to the support body between first and second positions. In the first position, the proximal end of the stylet is closer to the luer connector than in the second position.

The plunger assembly can also include a plunger comprising a first segment coupled to or defined by the stylet and a second segment having a greater cross-sectional size that the first segment. The second segment can merge into an external plunger flange. The support body can enclose the first segment and at least a portion of the second segment and the first segment and the second segment can be longitudinally movable relative to the support body. With the plunger in a first position, the stylet is in the first position and the second segment of the stylet resides closer to the luer connector than when the stylet is in the second position.

The plunger assembly can further include a drive screw that resides at least partially in the support body and that is coupled to the proximal end of the stylet.

The plunger assembly can further include a collar that is coupled to the support body and the drive screw. The collar can be rotatable in clockwise and counterclockwise directions to translate the stylet.

The plunger assembly can further include a support tube residing inside the support body and coupled to the luer connector above the seal. The stylet can be slidably coupled to the support tube to retract and extend inside the support tube while the support body slidably retracts and extends in concert with the stylet about an outer wall of the support tube.

Embodiments of the disclosure are directed to a surgical plunger assembly that includes: a stylet having opposing proximal and distal ends; a luer connector having an internal seal residing adjacent the proximal end of the stylet; a support body coupled to the luer connector and extending above the distal end of the stylet; and a plunger with a first segment coupled to or defined by the stylet and a second segment having a greater cross-sectional size that the first segment. The second segment merges into an external plunger flange. The support body encloses the first segment and at least a portion of the second segment and the first segment and the second segment are longitudinally movable relative to the support body. With the plunger in a first position, the second segment resides closer to the luer connector than in a second position.

The stylet can have a length outside the support body that is in a range of 6 inches and 10 feet in one or both of the first and second positions.

The stylet can have a length outside the support body that is in a range of 6 inches and 10 feet when the plunger is in each of the first and second positions.

The stylet can be formed of an MRI compatible material and can have a maximal outer diameter in a range of about 0.005 inches and about 0.020 inches.

The stylet can be formed of Nitinol.

The stylet can be formed of fused silica.

Other embodiments are directed to an intrabody fluid transfer system that includes: a cannula assembly with a proximal end with a luer connector and having a longitudinally opposing distal end with an open channel extending therethrough; and a plunger assembly coupled to the cannula assembly. The plunger assembly can have a stylet that extends in the open channel of the cannula assembly to position a distal end of the stylet adjacent the distal end of the cannula assembly. The open channel and the stylet cooperate to define a fluid channel extending from the distal end of the cannula assembly, optionally having a length in a range of about 1 cm to about 30 cm.

With the plunger in a first position associated with a ready to intake fluid or a fully injected position, the distal end of the stylet can extend flush with or out of the distal end of the cannula assembly.

Fluid can be held for dispensing to a patient in the fluid channel at a location between the distal end of the cannula assembly and a medial portion of the cannula assembly.

The plunger assembly can further include a luer connector with an internal seal residing adjacent the stylet. The luer connector of the plunger assembly can be attached to the luer connector of the cannula assembly. The plunger assembly can also include a support body coupled to the luer connector of the plunger assembly and extending above the distal end of the stylet and a plunger having a first segment coupled to or defined by the stylet and a second segment having a greater cross-sectional size that the first segment. The second segment can merge into an external plunger flange. The support body can enclose the first segment and at least a portion of the second segment and the first segment and the second segment can be longitudinally movable relative to the support body. With the plunger in a first position, the second segment can reside closer to the luer connectors than in a second position.

The intrabody fluid transfer assembly can further include a drive screw that resides at least partially in the support body and that is coupled to the proximal end of the stylet.

The intrabody fluid transfer assembly can further include a collar that is coupled to the support body and the drive screw. The collar can be rotatable in clockwise and counterclockwise directions to translate the stylet.

The intrabody fluid transfer assembly can further include a support tube residing inside the support body and coupled to the luer connector above the seal. The stylet can be slidably coupled to the support tube to retract and extend inside the support tube while the support body slidably retracts and extends in concert with the stylet about an outer wall of the support tube.

The stylet can have a length outside the support body that is in a range of 6 inches and 10 feet when the plunger is in each of the first and second positions.

The stylet can be formed of an MRI compatible material and can have a maximal outer diameter in a range of about 0.005 inches and about 0.020 inches.

The stylet can be formed of Nitinol.

The stylet can be formed of fused silica.

Other embodiments are directed to methods for transferring fluid into or from a subject. The methods include: providing a cannula assembly having a luer connector on a proximal end thereof and having a longitudinally opposing distal end; and providing a plunger assembly that is coupleable to or coupled to the cannula assembly. The plunger assembly has a stylet extending from the proximal end to a position proximate, flush with or beyond the distal end of the cannula assembly. The methods further include creating a vacuum by withdrawing the stylet relative to the distal end of the cannula assembly; and intaking target fluid into the distal end of the cannula assembly in response to the vacuum created by the stylet and the cannula assembly.

The methods can also include placing the cannula assembly with the plunger assembly coupled thereto and holding the fluid into a trajectory guide of a surgical navigation system whereby a proximal end portion of the plunger assembly is above the trajectory guide and the distal end of the cannula assembly and stylet are in a body of a patient and transferring the fluid into the patient.

The fluid can include stem cells.

The method can further include delivering the fluid to an intrabrain target site for the transferring step.

It is noted that aspects of the invention described with respect to one embodiment may be incorporated in a different embodiment although not specifically described relative thereto. That is, all embodiments and/or features of any embodiment can be combined in any way and/or combination. Applicant reserves the right to change any originally filed claim or file any new claim accordingly, including the right to be able to amend any originally filed claim to depend from and/or incorporate any feature of any other claim although not originally claimed in that manner. These and other objects and/or aspects of the present disclosure are explained in detail below.

### BRIEF DESCRIPTION OF THE DRAWINGS

**FIG. 1** is a top perspective view of an example cannula assembly according to embodiments of the present invention.
**FIG. 2** is a top view of an example plunger assembly according to embodiments of the present invention.
**FIG. 3** is an assembled view of the plunger and cannula assemblies shown in **FIGs. 1** and **2** forming a fluid transfer assembly according to embodiments of the present invention.
**FIG. 4** is an enlarged partial view of a proximal end portion of the assembled components shown in **FIG. 3****.**
**FIG. 5A** is a side perspective view of a proximal end portion of the cannula assembly shown in **FIG. 1** and a distal end of the plunger assembly shown in **FIG. 2** in a first assembly configuration.
**FIG. 5B** shows the proximal end portion of the cannula assembly shown in **FIG. 5A** with the plunger assembly shown in a further assembled state relative to **FIG. 5A****.**
**FIG. 6A** illustrates the luer connector of the cannula assembly of **FIG. 1** attached to the luer connector of the plunger assembly of **FIG. 2** after further insertion from the configuration shown in **FIG. 5B** according to embodiments of the present invention.
**FIG. 6B** is a greatly enlarged end perspective view of a distal end of the assembled device shown in **FIG. 3****.**
**FIG. 7** is a side perspective view of a proximal end portion of the assembled device shown in **FIG. 3** with the plunger in position for actuation for intaking fluid at the distal end thereof according to embodiments of the present invention.
**FIG. 8** is a side perspective view of the proximal end portion of the assembled device shown in **FIG. 7** with the plunger in a second position for actuation for dispensing/injecting fluid at the distal end thereof according to embodiments of the present invention.
**FIG. 9A** is an enlarged distal end perspective view of the assembled device shown when in the position of **FIG. 7****.**
**FIG. 9B** is an enlarged distal end perspective view of the assembled device shown when in the position of **FIG. 8****.**
**FIG. 10A** is an enlarged partial proximal end perspective view of another example of a plunger assembly according to embodiments of the present invention.
**FIG. 10B** is a partial section view of an internal interface of support tubing and plunger wire shown in the plunger assembly shown in **FIG. 10A** according to embodiments of the present invention.
**FIG. 10C** is a partial section view of the plunger assembly shown in **FIG. 10A** according to embodiments of the present invention.
**FIG. 11** is an enlarged partial perspective view of another embodiment of a plunger assembly according to embodiments of the present invention.
**FIG. 12** is a greatly enlarged section view of a segment of the proximal end portion of the plunger assembly shown in **FIG. 11****.**
**FIG. 13** is a schematic illustration of a medical intrabody fluid transfer system operable in an image guided surgical navigation system according to embodiments of the present disclosure.
**FIG. 14** is an enlarged partial section view of an example cannula and plunger assembly shown in **FIG. 3****,** held by a trajectory guide for intrabody placement according to embodiments of the present invention.
**FIG. 15** is a flow chart of exemplary actions that can be carried out according to embodiments of the present disclosure.

### DETAILED DESCRIPTION

The present invention now is described more fully hereinafter with reference to the accompanying drawings, in which some embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

Like numbers refer to like elements throughout. In the figures, the thickness of certain lines, layers, components, elements or features may be exaggerated for clarity. The terms "FIG." and "Fig." are used interchangeably with the word "Figure" in the specification and/or figures.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

It will be understood that when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being, for example, "directly on", "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

Spatially relative terms, such as "under," "below," "lower," "over," "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is inverted, elements described as "under" or "beneath" other elements or features would then be oriented "over" the other elements or features. Thus, the exemplary term "under" can encompass both an orientation of "over" and "under". The device may be otherwise oriented (rotated 90 degrees or at other orientations) and the spatially relative descriptors used herein interpreted accordingly. Similarly, the terms "upwardly," "downwardly," "vertical," "horizontal" and the like are used herein for the purpose of explanation only unless specifically indicated otherwise.

The term "about," as used herein with respect to a value or number, means that the value or number can vary by +/- twenty percent (20%).

The term "monolithic" means that the component (e.g., inner capillary tube or needle) is formed of a single uniform material.

The term "MRI visible" means that a device is visible, directly or indirectly, in an MRI image. The visibility may be indicated by the increased SNR of the MRI signal proximate to the device (the device can act as an MRI receive antenna to collect signal from local tissue) and/or that the device actually generates MRI signal itself, such as via suitable hydro-based coatings and/or fluid (typically aqueous solutions) filled channels or lumens.

The term "MRI compatible" means that a device is safe for use in an MRI environment and/or can operate as intended in an MRI environment without generating MR signal artifacts, and, as such, if residing within the high-field strength region of the magnetic field, is typically made of a non-ferromagnetic MRI compatible material(s) suitable to reside and/or operate in a high magnetic field environment.

The term "high-magnetic field" refers to field strengths above about 0.5T (Tesla), typically above 1.0T, and more typically between about 1.5T and 10T.

The term "near real time" refers to both low latency and high frame rate. Latency is generally measured as the time from when an event occurs to display of the event (total processing time). For tracking, the frame rate can range from between about 100 fps to the imaging frame rate. In some embodiments, the tracking is updated at the imaging frame rate. For near "real-time" imaging, the frame rate is typically between about 1 fps to about 20 fps, and in some embodiments, between about 3 fps to about 7 fps. The low latency required to be considered "near real time" is generally less than or equal to about 1 second. In some embodiments, the latency for tracking information is about 0.01s, and typically between about 0.25-0.5s when interleaved with imaging data. Thus, with respect to tracking, visualizations with the location, orientation and/or configuration of a known intrabody device can be updated with low latency between about 1 fps to about 100 fps. With respect to imaging, visualizations using near real time MR image data can be presented with a low latency, typically within between about 0.01 ms to less than about 1 second, and with a frame rate that is typically between about 1- 20 fps. Together, the system can use the tracking signal and image signal data to dynamically present anatomy and one or more intrabody devices in the visualization in near real-time. In some embodiments, the tracking signal data is obtained and the associated spatial coordinates are determined while the MR image data is obtained and the resultant visualization(s) with the intrabody device (e.g., stylet) and the near RT MR image(s) are generated.

The term "sterile," as used herein, means that a device, kit, and/or packaging meets or exceeds U.S./Federal Drug Administration and/or other regulatory medical/surgical cleanliness guidelines, and typically is free from live bacteria or other microorganisms.

Embodiments of the present invention can be utilized with various diagnostic or interventional devices and/or therapies to any desired internal region of an object using any suitable imaging modality, typically an MRI and/or in an MRI scanner or MRI interventional suite. However, CT or other imaging modalities may be used. The object can be any object, and may be particularly suitable for animal and/or human subjects for e.g., animal studies and/or veterinarian or human treatments. Some embodiments deliver therapies to the spine. Some embodiments deliver therapies to treat or stimulate a desired region of the sympathetic nerve chain. Other uses, inside or outside the brain, nervous system or spinal cord, include stem cell placement, gene therapy or drug delivery for treating physiological conditions, chemotherapy, drugs including replicating therapy drugs. Some embodiments can be used to treat tumors.

The term "substance," as used herein, refers to a liquid for treating or facilitating diagnosis of a condition and can include bions, stem cells or other target cells to site-specific regions in the body, such as neurological, nerves or other target sites and the like. In some embodiments, stem cells and/or other rebuilding cells or products can be delivered into spine, brain or cardiac tissue, such as a heart wall via a minimally invasive MRI guided procedure, while the heart is beating (i.e., not requiring a non-beating heart with the patient on a heart-lung machine). Examples of known stimulation treatments and/or target body regions are described in U.S. Patent Nos. 6,708,064; 6,438,423; 6,356,786; 6,526,318; 6,405,079; 6,167,311; 6539,263; 6,609,030 and 6,050,992.

The term "fluid" with respect to fluid being withdrawn from a subject refers to soft tissue, foreign matter, biological matter including cellular material and liquid in a subject.

The term "infusion" and derivatives thereof refers to the delivery of a substance (which can be a single substance or a mixture) at a relatively slow rate so that the substance can infuse about a target region. Thus, the term "infusate" refers to a substance so delivered.

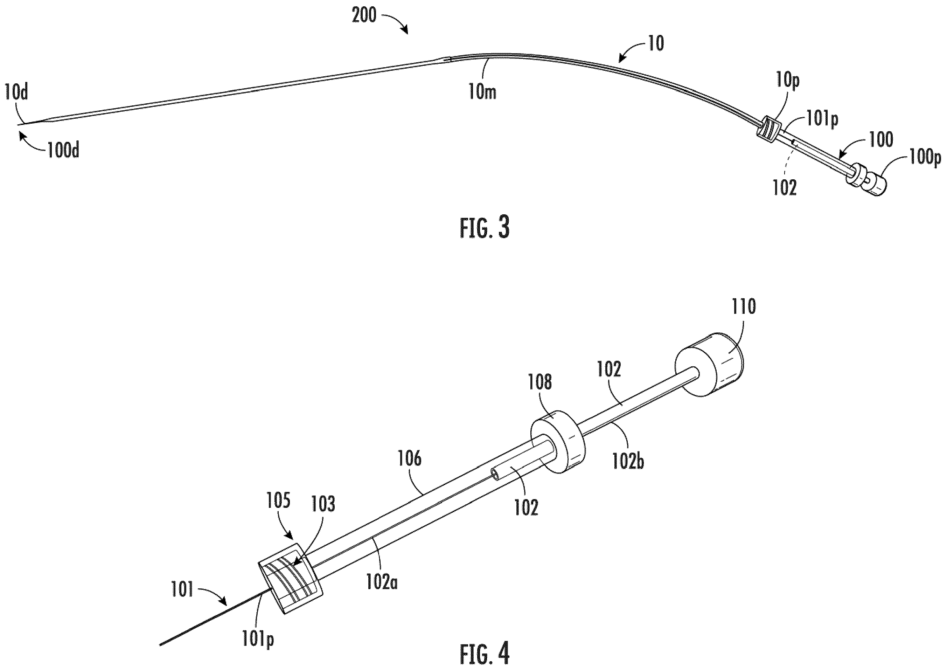

The term "semi-rigid" refers to devices that have sufficient rigidity to have a self-supporting fixed shape (typically straight tubular or cylindrical shapes) in the absence of applied bending forces but have sufficient flexibility to be able to bend or deflect without breaking in response to forces applied during insertion into or removal from a trajectory guide (see, for example, **1250t,** **FIG. 4****),** then return to its original self-supporting shape upon removal of the applied force(s).

The term "flexible" means that the device(s) does not have sufficient rigidity to have a fixed shape without support and can be rolled, coiled, folded for example.

The subject can be any subject, and may be particularly suitable for animal and/or human subjects for e.g., animal studies and/or veterinarian or human treatments.

Some embodiments aspirate fluid from a target intrabody region such as, for example, a brain. For example, aspiration of fluid from a target structure can debulk it. Debulking the structure can relieve pressure on the surrounding areas. This can be desirable as it can be performed in a less invasive manner than surgical resection. *See,* U.S. Patent Application Serial No. 16/217,222.

Embodiments of the invention can deliver therapies to the spine.

Embodiments of the invention can deliver therapies to treat or stimulate a desired region of the sympathetic nerve chain. Other uses, inside or outside the brain, nervous system or spinal cord, include stem cell placement, gene therapy or drug delivery for treating physiological conditions, chemotherapy, drugs including replicating therapy drugs. Some embodiments can be used to treat a patient with one or more tumors.

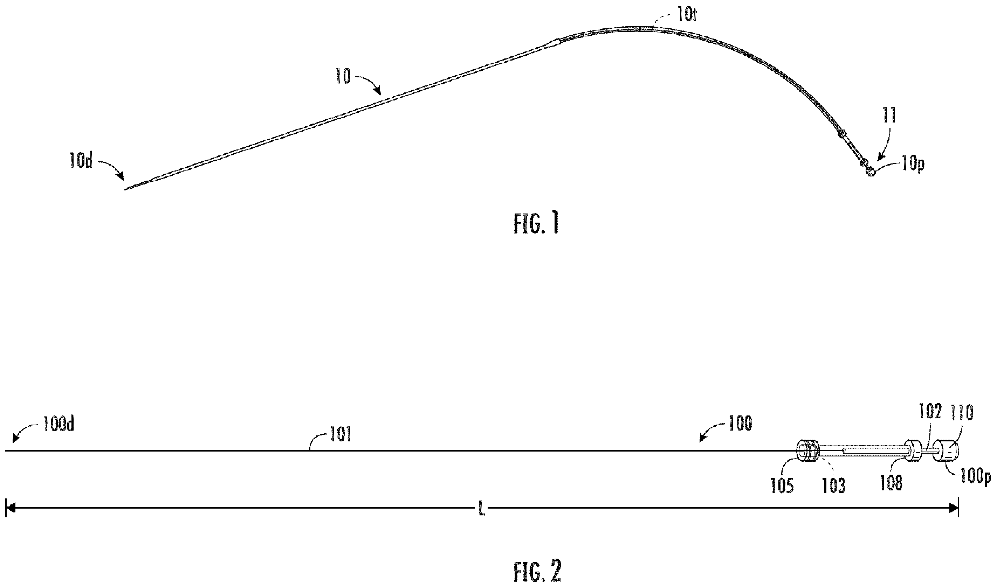

Embodiments of the present invention will now be described in further detail below with reference to the figures. **FIG. 1** illustrates an exemplary cannula assembly **10.** The cannula assembly **10** is configured for intrabody fluid transfer. **FIG. 2** illustrates an exemplary plunger assembly **100**. **FIG. 3** illustrates the plunger assembly **100** coupled to the cannula assembly **10** to define a transfer assembly **200.** When assembled, the plunger assembly **100** extends entirely through the cannula assembly **10.** A distal end **100d** of the plunger assembly **100** can reside proximate to, such as inward a distance of about 1 mm relative to the distal end **10d,** flush with and/or extend out beyond the distal end **10d** of the cannula assembly **10** and a proximal end **100p** of the plunger assembly **100** extends beyond a proximal end **10p** of the cannula assembly **10**. The distal end **100d** of the plunger assembly **100** can be configured to extend a short distance, such as less than 1 mm, typically such as a distance in a range of about 0-0.5 mm, beyond the distal end **10d** of the cannula assembly in a ready to load and/or fully dispensed position. The proximal end **10p** of the cannula assembly **10** can comprise a luer connector **11.**

In some embodiments, the plunger assembly **100** is configured to cooperate with the cannula assembly **10,** allowing the assembled device **200** to be "front loaded". The purpose of front loading the cannula assembly **10** is to load a target fluid into a delivery device to minimize and/or not create any "dead space". The term "dead space" refers to a situation where an undesirable amount of drug is left in a syringe or lumen of the cannula, after a delivery such as an infusion is complete. This can be particularly undesirable where a target drug for delivery is in limited supply such as comprising stem cells and/or where a cell sample obtained from a patient is very small.

Referring to **FIGs. 2-4****,** as shown, the plunger assembly **100** includes a support body **106** that can optionally be cylindrical (or other shape such as a polygonal or triangular shape) and comprises a luer hub **105** on a distal end thereof. The plunger assembly **100** can also include a plunger support flange **108** on an opposing proximal end.

The plunger assembly **100** can also include a plunger flange **110** that defines the proximal end **100p** of the plunger assembly **100**.

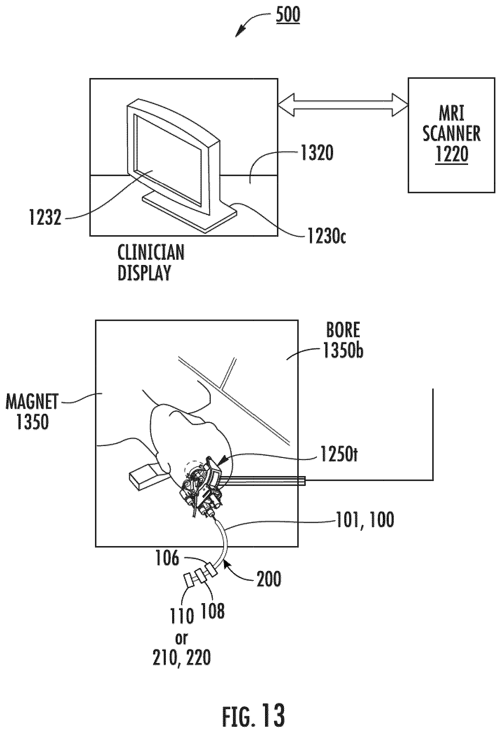

As is also shown, the plunger assembly **100** comprises a long stylet **101.** The long stylet **101** is elongate and has a length **L** (in a longitudinal direction) that is greater than a length of the support body **106.** Typically, the plunger cannula assembly **100** extends an overall length **L** that can be greater than an overall length **L** of the cannula assembly **10,** such as in a range of about 1 foot to about 10 feet including about 1.5 feet, about 2 feet, about 2.5 feet, about 3 feet, about 3.5 feet, about 4 feet, about 4.5 feet, about 5 feet, about 5.5 feet, about 6 feet, about 6.5 feet, about 7 feet, about 7.5 feet, about 8 feet, about 8.5 feet, about 9 feet, about 9.5 feet and about 10 feet. If sufficiently long, the plunger assembly **100** can be actuated from outside a bore **1350b** of a magnet **1350** of an MRI Scanner **1220** (**FIG. 13**) allowing efficient delivery during an image-guided surgical procedure.

The stylet 101 can be configured to fit any target inner diameter of a respective cannula assembly 10. The plunger assembly 100 can have sufficient flexibility to be able to change in shape from a linear configuration to a curved configuration as shown with respect to FIG. 2 and FIG. 3.

The cannula assembly 10 may have an inner diameter in a range of about 0.008 inches to about 0.021 inches, which may be particularly suitable for certain infusion uses.

When an operator pulls back on the plunger 102, the stylet 101 travels back as well creating a vacuum that evacuates/sucks a fluid such as a drug from a distal end 10d of cannula assembly 10 into the cannula assembly 10 about the stylet 101. Typically, the "front-loaded" drug resides upstream of the distal end **10d** of the cannula assembly **10** a distance corresponding to a stroke distance of the plunger **102** and/or a distance in a range of 1 cm to about 30 cm or a range of about 5 cm to about 15 cm. The distance can be, for example, about 1 cm, about 2 cm, about 3 cm, about 4 cm, about 5 cm, about 6 cm, about 7 cm, about 8 cm, about 9 cm, about 10 cm, about 11 cm, about 12 cm, about 13 cm, about 14 cm, about 15 cm, about 16 cm, about 17 cm, about 18 cm, about 19 cm, about 20 cm, about 21 cm, about 22 cm, about 23 cm, about 24 cm, about 25 cm, about 26 cm, about 27 cm, about 28 cm, about 29 cm and about 30 cm. The stroke travel distance is below the luer connector **11** and/or luer hub **105** and/or support body **106,** more typically between the distal end **10d** of the cannula assembly **10** and a medial location **10m** of the cannula assembly **10.**

When the plunger **102** is moved forward toward the distal end **10d** of the cannula assembly **10,** the stylet **101** pushes the fluid, e.g., drug, out of the cannula assembly **10** into a target location such as the brain or heart, for example.

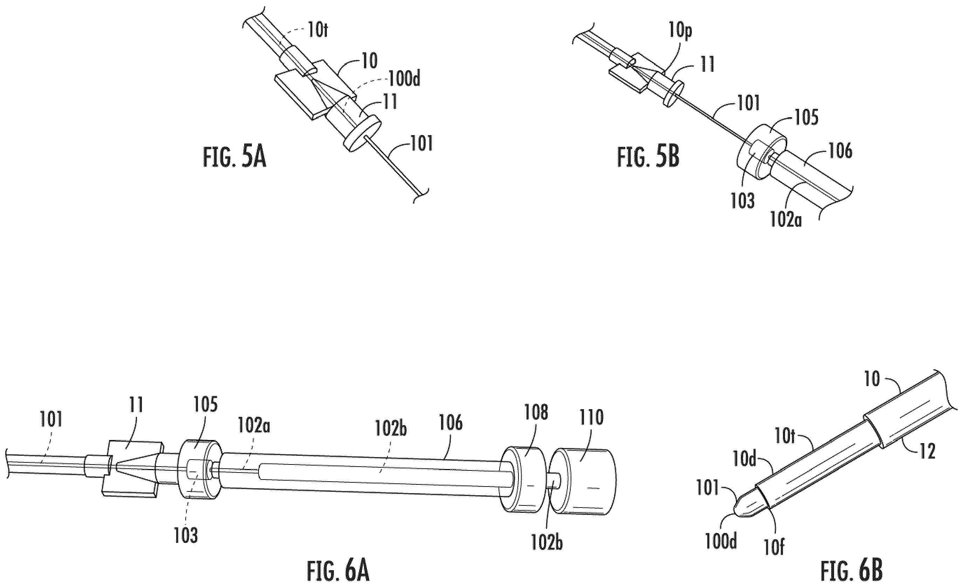

Referring to **FIGs. 5A, 5B, 6A** and **6B****,** the plunger assembly **100** can attach to the cannula assembly **10** by inserting the stylet **101** into the proximal end **10p** of the cannula assembly **10** and threading/pushing the stylet **101** forward to the distal end **10d** of the cannula assembly **10,** then the plunger assembly **100** can be coupled to (typically locked onto) the cannula assembly **10** at the luer connector **11** (**FIG. 6A**), similar to a syringe. When fully seated, the distal end **100d** of the plunger assembly **100** can reside at a distal end **10d** of the cannula assembly **10,** and can protrude a distance in a range of about 0-0.5 mm (**FIG. 6B**) in some particular embodiments.

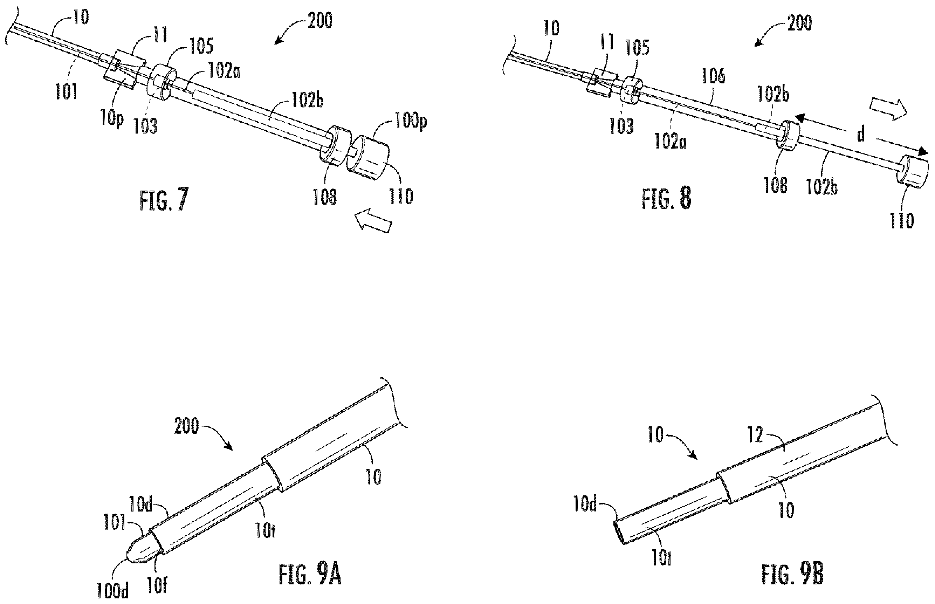

**FIG. 7** illustrates the plunger/cannula assembly **200** in a ready to intake configuration with the plunger flange **110** adjacent the support body flange **108** and the first segment **102a** of the plunger **102** having a shorter length inside the support body **106** than the position shown in **FIG. 8**, with the second segment **102b** residing closer to the seal **103** and luer hub **105** in the ready to intake configuration: **FIGs. 7, 9A** versus the retracted and loaded position shown in **FIGs. 8**; **9B** (the latter having the stylet **101** retracted from the distal end **10d** of the cannula assembly).

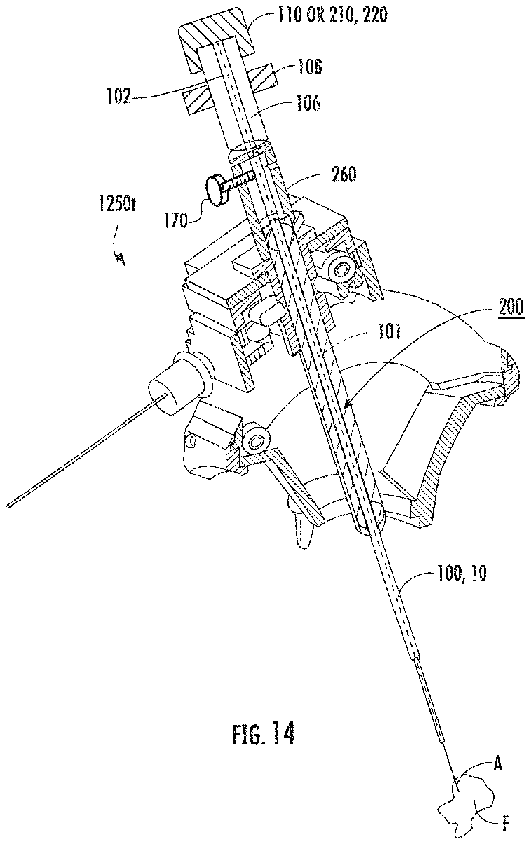

When in the configuration shown in **FIG. 8**, the assembly **200** can be inserted through a stereotactic guidance system such as a trajectory guide **1250t** (**FIG. 14**) with the plunger flange **110** accessible to move up and down relative to a patient and the fluid **F** delivered to or obtained from a patient using the assembly **200.**

The plunger assembly **100** can be provided in a kit assembled to the cannula assembly **10.**

The plunger assembly **100** can be provided as a separate component in a package with or in a different package from the cannula assembly **10.**

The drug can be provided separate from the plunger assembly **10** and/or cannula assembly **100** and is typically loaded onsite prior to delivery.

In some embodiments, for MRI conditional use (safe for use about an MRI Scanner room with magnets generating a magnetic field, but cannot be inserted during active MRI scanning), the stylet **101** can comprise Nitinol wire. In some embodiments, for MRI-safe use (safe for use in an MRI Scanner room even during scanning), the stylet **101** can comprise fused silica.

In some embodiments, for CT or other imaging modalities other materials may be used, preferably biocompatible and inert with respect to the target drug or fluid.

The stylet **101** defines or is attached to a plunger **102.** The plunger **102** and stylet **101** are longitudinally extendable and retractable (as a unit/in concert) relative to the cannula assembly **10** and/or a luer hub **105** attached to the plunger assembly **100.**

An internal seal **103** can reside at the luer hub **105.** The internal seal **103** can define a fluid-tight seal about a segment of the stylet **101** defining a segment of the plunger **102w.** The segment of the plunger defined by the stylet **101** can be a small diameter wire **102w.**

In some embodiments, at least an elongate segment of the stylet **101** that resides in a distal and medial (intrabody) portion of the cannula assembly 10 can be solid (have a solid core) and have an outer diameter in a range of about 0.005 inches and about 0.020 inches.

The plunger **102** can comprise a first segment **102a** that merges into a longitudinally extending second segment **102b.** The second segment **102b** can have a greater cross-sectional size relative to the first segment **102a** inside a support body **106.** The second segment **102b** can snugly slidably engage the support body **106** but is not required to be fluidly sealed thereto. The support body **106** can be configured to support the plunger **102** including at least part of segments **102a, 102b** during actuation to inhibit buckling.

At least a portion of the first segment **102a** and a portion of the second segment **102b** can reside in the support body **106** when in a fully extended position with the distal end **100d** outside the distal end **10d** of the cannula assembly (**FIGs. 7, 9A**) and when in a retracted position for loading/intaking fluid (**FIGs. 8, 9B**). Thus, the second segment **102b** can travel longitudinally with respect to the support body **106** but does not travel below the internal seal **103** and/or luer hub **105** (**FIG. 7**). The stroke distance "**d**", between the maximal extended and retracted operative positions, e.g., a location to intake/load fluid relative to a fully assembled position (**FIG. 7**), can be a distance in a range of about 1 cm to about 30 cm or a range of about 5 cm to about 15 cm, depending on a desired amount of fluid to be loaded into the flow channel **10f** of the assembled device **200** (**FIGs. 3****,** **7, 8**). The flow channel **10f** may have an annular segment defined by the stylet and cannula assembly. The stroke distance can be, for example, about 1 cm, about 2 cm, about 3 cm, about 4 cm, about 5 cm, about 6 cm, about 7 cm, about 8 cm, about 9 cm, about 10 cm, about 11 cm, about 12 cm, about 13 cm, about 14 cm, about 15 cm, about 16 cm, about 17 cm, about 18 cm, about 19 cm, about 20 cm, about 21 cm, about 22 cm, about 23 cm, about 24 cm, about 25 cm, about 26 cm, about 27 cm, about 28 cm, about 29 cm and about 30 cm.

The distal end of the stylet **101** can travel 1:1 with the plunger **102** proximal end and pull fluid in to the cannula **10** in a 1:1 ratio, e.g., 1 cm of plunger travel can intake fluid a distance of 1 cm.

The stylet **101** and the cannula assembly **10** can be sized and configured to create a vacuum and intake the fluid using the vacuum to front load the cannula assembly 10. The differential between the inner diameter of the cannula **10** and the outer diameter of the stylet **101** to create a desirable vacuum can be in arrange of about 0.0005 inches and 0.10 inches, for example.

The cannula assembly **10** can comprise an inner tube **10t** of continuous length and may have a constant inner diameter, at least over a major portion of a length thereof. In some embodiments, the inner tube can be one continuous piece of material, typically of either PEEK or fused silica glass that extends from the distal end **10d** to the connector **11.**

Referring to **FIG. 1**, the stylet **101** can cooperate with the inner tube **10t** and define a flow channel **10f** (**FIG**. **9A**). The flow channel **10f** can have an outer diameter that can be in a range about 0.005 inches and about 0.020 inches, in a range of 100 µm to about 750 µm, such as about 200 µm, or in a range of about 0.20 mm to about .05 mm, such as about 0.20 mm or about 0.053 mm.

The inner tube **10t** can comprise fused silica glass or PEEK or other material. The stylet **101** and inner tube **10t** can be substantially, if not totally, inert and sterile, so as not to chemically interact with any target fluid in the flow lumen **10f** (**FIG. 9A**).

Referring to **FIGs. 1****,** **6B****,** **9B****,** the cannula assembly **10** can comprise an outer tube 12 that can be closely spaced to the inner surface of the outer wall of the inner tube 1**0t** to inhibit reverse flow and/or provide a fluid-resistant interface to inhibit flow therebetween.

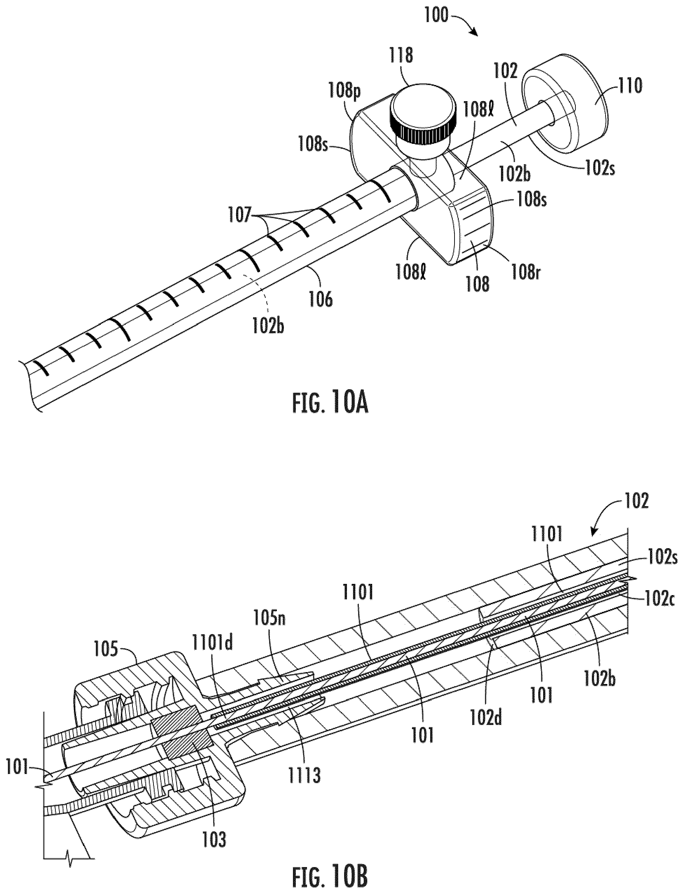

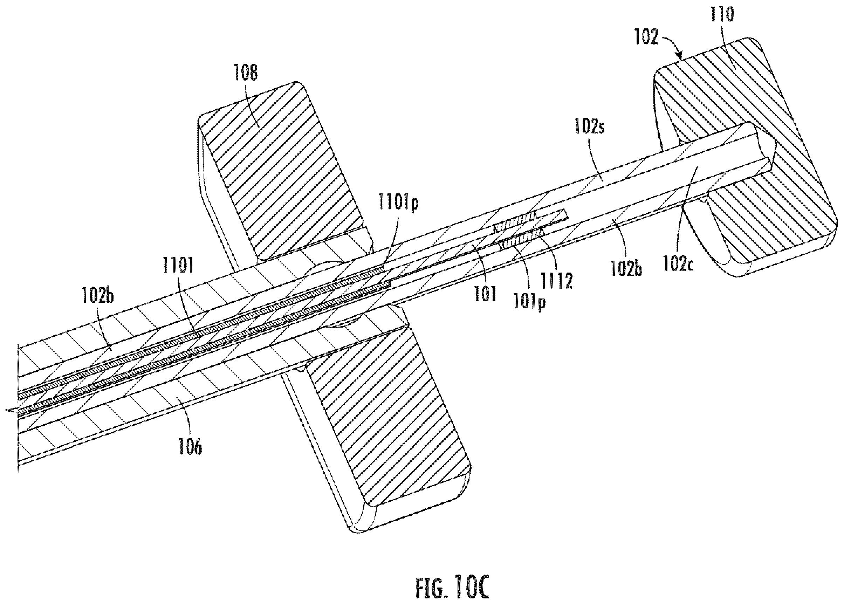

Referring to **FIG. 10A****,** the plunger assembly **100** can comprise graduated indicia of depth/distance on the support body **106.** The support flange **108** can also include a flange **108** that has an outer perimeter **108p** with long sides **108l** connected by shorter sides **108s.** The shorter sides **108s** can define finger gripping surfaces. A lock member **118** such as a thumbscrew can be provided on at least one of the long sides **108.** The shorter sides **108s** can be arcuate while the longer sides may be planar. The shorter sides **108s** can comprise ridges **108r.** The lock member **118** can be configured to directly couple to an outer surface **102s** of the second segment **102b** of the plunger **102** inside the support body **106.** **FIGs. 10B** and **10C** illustrate that the second segment **102b** of the plunger **102** can comprise a sleeve **102s** with a longitudinally extending channel **102c.** The channel **102c** can extend above a proximal end portion **101p** of the stylet **101.** The proximal end portion **101p** of the stylet **101** can be affixed to the channel **102c** so that the sleeve **102s** (plunger wire outer body) and style **101** slide in concert.

**FIGs 10B** and **10c** illustrate that the plunger assembly **100** can further include a support tube **1101** that resides between the seal **103** of the luer hub **105** and the plunger flange **110** and encases a portion of the stylet **101.** The support tube **1101** can have a distal end portion **1101d** that resides in an inwardly extending neck **105** of the luer hub **105,** axially aligned with the channel **102c** of the sleeve **102.**

As shown in **FIG. 10C****,** a proximal end portion **1101p** of the support tube **1101** can terminate proximate an outer end of the support flange **108.** The support tube **1101** can reside inside the sleeve **102s** and can terminate short of the proximal end portion **101p** of the stylet **101.** The proximal end portion **101p** of the style **101** can be affixed to the plunger flange **110** via a bond **1112** inside the channel **102c** under the flange **110.**

The stylet **101** can be configured to slide longitudinally inside the support tube **1101** and the sleeve **102** can slide longitudinally outside the support tube **1101.** The support tube **1101** can have a stationary configuration inside the support body **106.** At least one end portion of the support tube **1101** can be affixed, such as via a bond **1113,** to the support tube **106,** shown as indirectly attached to the support tube **106,** to anchor the support tube **1101** in a fixed position in the plunger assembly **100.**

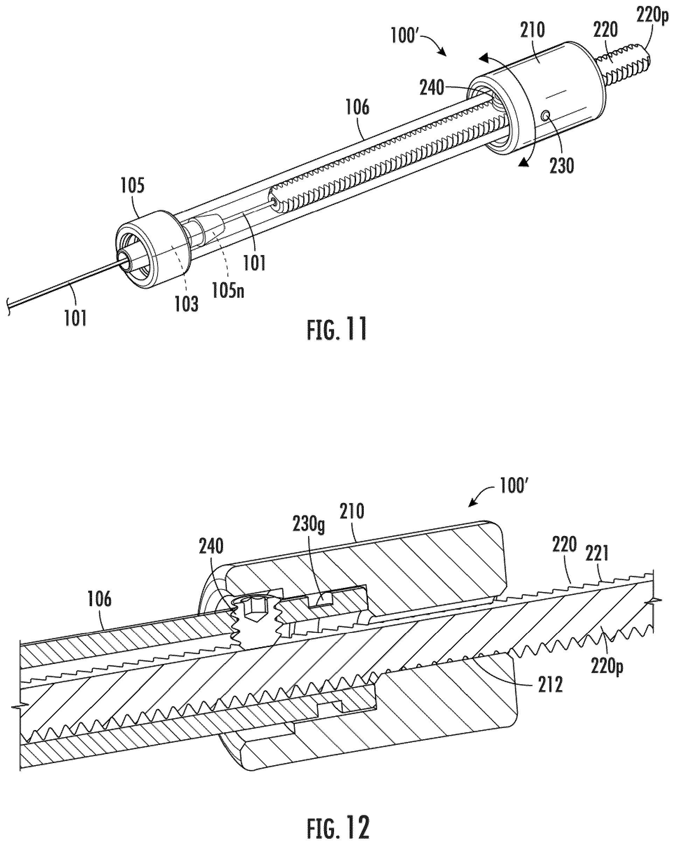

Turning now to **FIGs. 11** and **12**, another embodiment of a plunger assembly **100'** is shown. In this embodiment, a drive screw **220** can be used to translate the stylet **101** instead of a plunger **102,** which may provide for increased precision over manual plunger operation using the plunger flange. The stylet **101** can be attached to the drive screw **220** inside the support body **106,** above the luer hub **105** with the seal **103.** The plunger assembly **100'** can include a collar **210** that is affixed to the screw **220.** Rotation of the collar **210** can translate the drive screw **220,** which retracts or extends the stylet **101**.

One full rotation of the collar **210** can translate the stylet **101** a defined distance, such as a distance in a range of about 0.1 mm and about 2 mm. In some embodiments, one rotation of the collar **210** can be configured to provide about 1 mm of longitudinal travel (retraction and extension) of the stylet **101.**

The stylet **101** can be affixed to the drive screw **220.** The proximal end portion **101p** of the stylet can extend into a channel in the screw **220.** The proximal end portion **101p** of the stylet **101** can be bonded to the screw **220.**

The collar **210** can be fixed to the support body **106.** A set screw **240** can thread into the support body **106.** The set screw 240 can prevent the drive screw **220** from rotating inside the collar **210.**

A dowel pin **230** can engage an interior groove **230g** in the collar **210** to keep the collar **210** assembled to the support body **106** and/or prevent the drive screw **220** from rotating. The collar **210** can have internal threads **212** that engage the threads **221** of the drive screw **220.**

A proximal end portion **220p** of the drive screw **220** can extend out of the collar **210.** The threads **212** can extend over a sub-length of the collar **210** inside the collar **210.**

Although not shown, the support tube **1101** discussed above, can be provided in the support body **106,** under the drive screw **220.**

Referring to **FIG. 14****,** the assembly **200** can extend through a tubular support **260** of a trajectory guide **1250t** that can be held by a base or frame e.g., a stereotactic frame that can be secured to the patient or that can be secured to a holder residing over the patient. A lock **170** can be used to secure the assembly **200** at a desired position in the tubular support **260** to place the distal end **10d** at a target region **A** and withdraw or delivery substance **F.** *See, e.g.,* U.S. Patent Nos. 8,315,689, 8,175,677 and 8,374,677 and US Patent Application Publication No. 2010/0198052 (Serial No. 12/694,865) for descriptions of patient planning and entry protocols and frames and trajectory guides.

**FIG. 13** illustrates an MRI-guided interventional system **500** with an MRI scanner **1220**, a clinician workstation **1230** with at least one circuit **1230c**, at least one display **1232,** an MRI compatible trajectory guide **1250t** and a fluid transfer assembly **200.** The system **500** can be configured to render or generate near real time or real time visualizations of the target anatomical space using MRI image data and predefined data of at least one surgical tool (e.g., tubular cannula **20** and/or trajectory guide **1250t**) to segment the image data and place the trajectory guide **1250t** and the cannula **20** in the rendered visualization in the correct orientation and position in 3D space (which is the MRI surgical space for MRI embodiments), anatomically registered to a patient. The trajectory guide **1250t** and the cannula **20** can include or cooperate with tracking, monitoring and/or other interventional components.

The trajectory guide **1250t** can be configured to provide one or more of an X-Y adjustment and/or pitch and roll adjustment in order to accurately position the assembly **200** at a desired location within a patient. For additional discussion of examples of suitable trajectory guides, see U.S. Patent No. 8,374,677. However, it is noted that other trajectory guide configurations may be used and embodiments of the disclosure are not limited by the examples of the trajectory guides herein.

According to some embodiments, the systems are configured to provide a substantially automated or semi-automated and relatively easy-to-use MRI-guided system with defined workflow steps and interactive visualizations. In particular embodiments, the systems define and present workflow with discrete steps for finding target and entry point(s), guiding the alignment of the targeting cannula to a planned trajectory, monitoring the insertion of the cannula assembly **200,** and adjusting the (X-Y) position in cases where the placement needs to be corrected. During steps where specific MR scans are used, the circuit or computer module can display data for scan plane center and angulation to be entered at the console. The workstation/circuit can passively or actively communicate with the MR scanner. The system can also be configured to use functional patient data (e.g., fiber tracks, fMRI and the like) to help plan or refine a target surgical site and/or access path.

The system **500** may also include a decoupling/tuning circuit that allows the system to cooperate with the MRI scanner **1220** and filters and the like. *See, e.g.,* U.S. Patent Nos. 6,701,176; 6,904,307 and U.S. Patent Application Publication No. 2003/0050557.

The assembly **200** can be configured to flowably introduce, infuse and/or inject a desired therapy substance (e.g., antigen, gene therapy, chemotherapy or stem-cell or other therapy type).

In some embodiments, the intrabody fluid transfer assembly **200** is configured to deliver a drug therapy to the brain. The drug therapy can comprise substance **F** delivered to the target site or region **A** (**FIG. 14**) through the fluid/flow channel **10f** (**FIG. 6B**) and may be any suitable and desired substance for drug discovery, animal or human clinical trials and/or approved medical procedures. According to some embodiments, the substance **F** is a liquid or slurry. In the case of a tumor, the substance may be a chemotherapeutic (cytotoxic) fluid. In some embodiments, the substance can include certain types of advantageous cells that act as vaccines or other medicaments (for example, antigen presenting cells such as dendritic cells). The dendritic cells may be pulsed with one or more antigens and/or with RNA encoding one or more antigen. Exemplary antigens are tumor-specific or pathogen-specific antigens. Examples of tumor-specific antigens include, but are not limited to, antigens from tumors such as renal cell tumors, melanoma, leukemia, myeloma, breast cancer, prostate cancer, ovarian cancer, lung cancer and bladder cancer. Examples of pathogen-specific antigens include, but are not limited to, antigens specific for HIV or HCV. In some embodiments, the substance **F** may comprise radioactive material such as radioactive seeds. Substances **F** delivered to a target area in accordance with embodiments of the present invention may include, but are not limited to, the following drugs (including any combinations thereof) listed in **Table 1**:

**Table 1**

| **DRUG (generic name)** | **DISORDER(S)** |
|---|---|
| Caprylidene | Alzheimer's disease |
| Donepezil | Alzheimer's disease |
| Galantamine | Alzheimer's disease |
| Memantine | Alzheimer's disease |
| Tacrine | Alzheimer's disease |
| vitamin E | Alzheimer's disease |
| ergoloid mesylates | Alzheimer's disease |
| Riluzole | Amyotrophic lateral sclerosis |
| Metoprolol | Benign essential tremors |
| Primidone | Benign essential tremors |
| Propanolol | Benign essential tremors |
| Gabapentin | Benign essential tremors & Epilepsy |
| Nadolol | Benign essential tremors & Parkinson's disease |
| Zonisamide | Benign essential tremors & Parkinson's disease |
| Carmustine | Brain tumor |
| Lomustine | Brain tumor |
| Methotrexate | Brain tumor |
| Cisplatin | Brain tumor & Neuroblastoma |
| Ioversol | Cerebral arteriography |
| Mannitol | Cerebral Edema |
| Dexamethasone | Cerebral Edema & Neurosarcoidosis |
| Baclofen | Cerebral spasticity |
| Ticlopidine | Cerebral thrombosis / embolism |
| Isoxsuprine | Cerebrovascular insufficiency |
| Cefotaxime | CNS infection & Meningitis |
| Acyclovir | Encephalitis |
| Foscarnet | Encephalitis |
| Ganciclovir | Encephalitis |
| interferon alpha-2a | Encephalitis |
| Carbamazepine | Epilepsy |
| Clonazepam | Epilepsy |
| Diazepam | Epilepsy |
| divalproex sodium | Epilepsy |
| Ethosuximide | Epilepsy |
| Ethotoin | Epilepsy |
| Felbamate | Epilepsy |
| Fosphenytoin | Epilepsy |
| Levetiracetam | Epilepsy |
| Mephobarbital | Epilepsy |
| Paramethadione | Epilepsy |
| Phenytoin | Epilepsy |
| Trimethadione | Epilepsy |
| Pregabalin | Epilepsy & Neuralgia |
| immune globulin intravenous | Guillain-Barre Syndrome |
| interferon beta-1b | Guillain-Barre Syndrome & Multiple sclerosis |
| | Guillain-Barre Syndrome & Multiple sclerosis & |
| Azathioprine | Neurosarcoidosis |
| Risperidone | Head injury |
| Tetrabenazine | Huntington's disease |
| Acetazolamide | Hydrocephalus & Epilepsy |
| Alteplase | Ischemic stroke |
| Clopidogrel | Ischemic stroke |
| Nimodipine | Ischemic stroke & Subarachnoid hemorrhage |
| Aspirin | Ischemic stroke & Thromboembolic stroke |
| Amikacin | Encaphalitis |
| Ampicillin | Encaphalitis |
| ampicillin/sulbactam | Encaphalitis |
| Ceftazidime | Encaphalitis |
| Ceftizoxime | Encaphalitis |
| Cefuroxime | Encaphalitis |
| Chloramphenicol | Encaphalitis |
| cilastatin/imipenem | Encaphalitis |
| Gentamicin | Encaphalitis |
| Meropenem | Encaphalitis |
| Metronidazole | Encaphalitis |
| Nafcillin | Encaphalitis |
| Oxacillin | Encaphalitis |
| Piperacillin | Encaphalitis |
| Rifampin | Encaphalitis |
| sulfamethoxazole/trimethoprim | Encaphalitis |
| Tobramycin | Encaphalitis |
| Triamcinolone | Encaphalitis |
| Vancomycin | Encaphalitis |
| Ceftriaxone | Encaphalitis & Neurosyphilis |
| Penicillin | Encaphalitis & Neurosyphilis |
| Corticotrophin | Multiple sclerosis |
| Dalfampridine | Multiple sclerosis |
| Glatiramer | Multiple sclerosis |
| Mitoxantrone | Multiple sclerosis |
| Natalizumab | Multiple sclerosis |
| Modafinil | Multiple sclerosis |
| Cyclophosphamide | Multiple sclerosis & Brain tumor & Neuroblastoma |
| interferon beta-1a | Multiple sclerosis & Neuritis |
| Prednisolone | Multiple sclerosis & Neurosarcoidosis |
| Prednisone | Multiple sclerosis & Neurosarcoidosis |
| Amantadine | Multiple sclerosis & Parkinson's disease |
| Methylprednisolone | Neuralgia |
| Desvenlafaxine | Neuralgia |
| Nortriptyline | Neuralgia |
| Doxorubicin | Neuroblastoma |
| Vincristine | Neuroblastoma |
| Albendazole | Neurocystecercosis |
| chloroquine phosphate | Neurosarcoidosis |
| Hydroxychloroquine | Neurosarcoidosis |
| Infliximab | Neurosarcoidosis |
| Pentoxyfilline | Neurosarcoidosis |
| Thalidomide | Neurosarcoidosis |
| Apomorphine | Parkinson's disease |
| Belladonna | Parkinson's disease |
| Benztropine | Parkinson's disease |
| Biperiden | Parkinson's disease |
| Bromocriptine | Parkinson's disease |
| Carbidopa | Parkinson's disease |
| carbidopa/entacapone/levodopa | Parkinson's disease |
| carbidopa/levodopa | Parkinson's disease |
| Entacapone | Parkinson's disease |
| Levodopa | Parkinson's disease |
| pergolide mesylate | Parkinson's disease |
| Pramipexole | Parkinson's disease |
| Procyclidine | Parkinson's disease |
| Rasagiline | Parkinson's disease |
| Ropinirole | Parkinson's disease |
| Rotiotine | Parkinson's disease |
| Scopolamine | Parkinson's disease |
| Tolcapone | Parkinson's disease |
| Trihexyphenidyl | Parkinson's disease |
| Seleginline | Parkinson's disease |
| Rivastigmine | Parkinson's disease & Alzheimer's disease |
| Anisindione | Thromboembolic stroke |
| Warfarin | Thromboembolic stroke |
| 5-hydroxytryptophan | Depression & Anxiety & ADHD |
| Duloxetine | Depression & Anxiety & Bipolar disorder |
| Escitalopram | Depression & Anxiety & Bipolar disorder |
| | Depression & Anxiety & Bipolar disorder & Autism & |
| Venlafaxine | Social anxiety disorder |
| Desvenlafaxine | Depression & Anxiety & PTSD & ADHD |
| Paroxetine | Depression & Anxiety & PTSD & Social anxiety disorder |
| fluoxetine/olanzapine | Depression & Bipolar disorder |
| l-methylfolate | Depression & BPD |
| Amitriptyline | Depression & PTSD |
| Sertraline | Depression & PTSD & Bipolar disorder & Social anxiety disorder |
| Fluvoxamine | Depression & PTSD & Social anxiety disorder |
| Olanzapine | Depression & Schizophrenia & Bipolar disorder |
| Paliperidone | Depression & Schizophrenia & Bipolar disorder |
| Aripiprazole | Depression & Schizophrenia & Bipolar disorder & Autism |
| Quetiapine | Depression & Schizophrenia & PTSD & BPD & Bipolar disorder |
| Risperidone | Depression & Schizophrenia & PTSD & BPD & Bipolar disorder & Autism |
| Amisulpride | Depression & Social anxiety disorder |
| Chlorpromazine | Psychosis |
| Droperidol | Psychosis |
| Fluphenazine | Psychosis |
| Periciazine | Psychosis |
| Perphenazine | Psychosis |
| Thiothixene | Psychosis |
| Triflupromazine | Psychosis |
| Haloperidol | Psychosis & Dementia |
| Prazosin | PTSD |
| Clozapine | Schizophrenia |
| Flupenthixol | Schizophrenia |
| Iloperidone | Schizophrenia |
| Loxapine | Schizophrenia |
| Mesoridazine | Schizophrenia |
| Promazine | Schizophrenia |
| Reserpine | Schizophrenia |
| Thioridazein | Schizophrenia |
| Zuclopenthixol | Schizophrenia |
| Asenapine | Schizophrenia & Bipolar disorder |
| Levomepromazine | Schizophrenia & Bipolar disorder |
| Ziprasidone | Schizophrenia & Bipolar disorder |
| Molindone | Schizophrenia & Psychosis |
| Pimozide | Schizophrenia & Psychosis |
| Thioridazine | Schizophrenia & Psychosis |
| Cytarabine | Chemotherapy, hematological malignancies |

According to some embodiments, the intrabody fluid transfer system **200** can be configured as an infusate delivery system that is delivered to a patient at an infusion rate in the range of from about 1 µL/minute to about 3 µL/minute.

Insertion of the surgical cannula assembly **200** (or any other surgical, e.g., delivery, cannula) can be tracked in near real time by reference to a void in the patient tissue caused by the surgical cannula assembly **200** and reflected in the MR image. In some embodiments, one or more MRI-visible fiducial markers may be provided on the surgical tubular cannula **10,** MR scanned and processed, and displayed on the UI. In some embodiments, components of the surgical cannula assembly **200** may itself be formed of an MRI-visible material, MR scanned and processed, and displayed on the UI.

According to some embodiments, the surgical cannula/plunger assembly **200** may include an embedded intrabody MRI antenna (not shown) that is configured to pick-up MRI signals in local tissue during an MRI procedure. The MRI antenna can be configured to reside on a distal end portion of the surgical cannula. In some embodiments, the antenna has a focal length or signal-receiving length of between about 1-5 cm, and typically is configured to have a viewing length to receive MRI signals from local tissue of between about 1-2.5 cm. The MRI antenna can be formed as comprising a coaxial and/or triaxial antenna. However, other antenna configurations can be used, such as, for example, a whip antenna, a coil antenna, a loopless antenna, and/or a looped antenna. *See, e.g.,* U.S. Patent Nos. 5,699,801; 5,928,145; 6,263,229; 6,606,513; 6,628,980; 6,284,971; 6,675,033; and 6,701,176. *See also* U.S. Patent Application Publication Nos. 2003/0050557; 2004/0046557; and 2003/0028095.

While the devices have been described by way of example as delivery devices and exemplary methods for delivering a substance to a patient, in accordance with some embodiments the devices can be used to withdraw and/or aspirate a substance (e.g., spinal fluid, cardiac fluid or neuro fluid) from a patient. Thus, it will be appreciated that the devices and exemplary methods as disclosed herein can be used to transfer a substance into and/or from a patient.

While the devices have been described herein primarily with reference to MRI-guided insertion and infusion procedures, in some embodiments the devices can be used in procedures without MRI guidance, such as using other imaging modalities, such as, but not limited to, CT imaging systems, where image-guided surgical navigation is desired.

While the cannula/plunger assembly **200** has been described with the surgical assembly **200** coupled to a trajectory guide **1250t,** other types of trajectory guidance or stereotactic frames or without a stereotactic frame or trajectory guide.

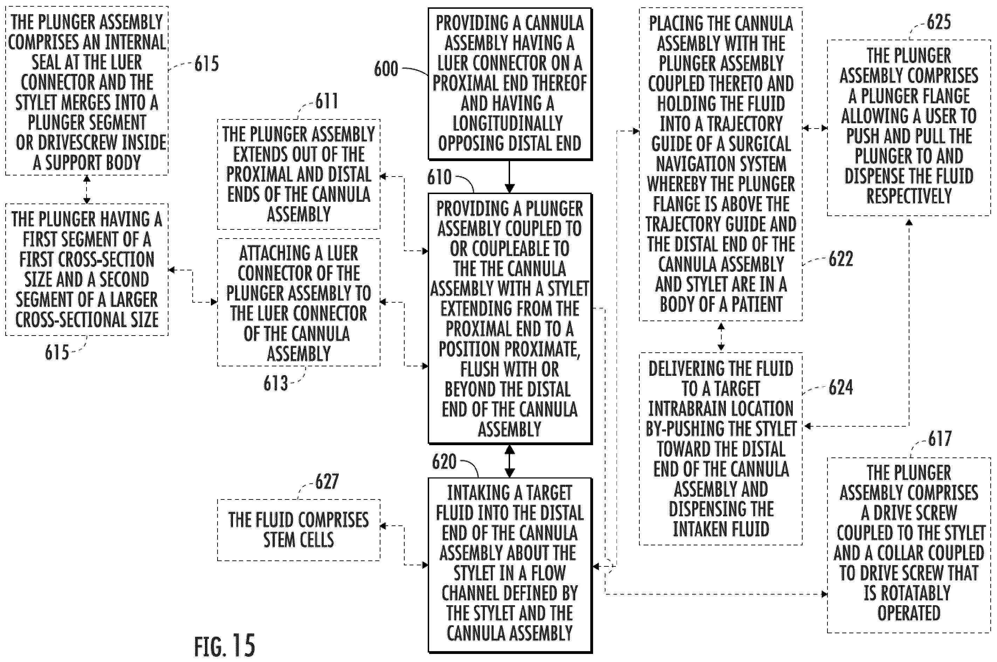

**FIG. 15** is a flow chart of exemplary actions that can be carried out according to embodiments of the disclosure. A cannula assembly having a luer connector on a proximal end thereof and having a longitudinally opposing distal end is provided (block **600**). A plunger assembly that is coupleable to or coupled to the cannula assembly is provided. The plunger assembly comprises a stylet extending from the proximal end to a position proximate, flush with or beyond the distal end of the cannula assembly (block **610**). A target fluid is intaken into the distal end of the cannula assembly about the stylet in a fluid/flow channel created/defined by the stylet and the cannula assembly (block **620**). The cannula assembly and the plunger assembly can cooperate to generate a vacuum that causes the fluid to be intaken into the distal end portion and upstream thereof (but typically below the plunger luer hub).

The plunger assembly can comprise a drive screw residing at least partially inside a support body and coupled to the stylet and a collar rotatably coupled to the drive screw configured to retract and extend the stylet (block **617**).

The cannula assembly with the plunger assembly coupled thereto and holding the fluid can be placed into a trajectory guide of a surgical navigation system whereby the plunger flange is above the trajectory guide and the distal end of the cannula assembly and stylet are in a body of a patient (block **622**).

The fluid can be delivered to a target intrabrain location by pushing a plunger toward the distal end of the cannula assembly and dispensing the intaken fluid (block **624**).

The plunger assembly comprises a plunger flange allowing a user to push and pull the plunger to intake and dispense the fluid (block **625**).

The plunger assembly is configured to extend to (concurrently) extend out of the proximal and distal ends of the cannula assembly (block **611**) (during at least the delivery/dispensing operation).

A luer connector of the plunger assembly can be connected to the luer connector of the cannula assembly (block **613**).

The plunger assembly comprises an internal seal at the luer connector and the stylet merges into a plunger segment or drive screw inside a support body (block **614**).

The plunger segment can have a first segment of a first cross-section size and a second segment of a larger cross-sectional size, optionally the first segment is a stylet and the second segment can be defined by a sleeve affixed to the stylet (block **615**).

The fluid comprises stem cells (block **627**).

The scope of the invention is defined by the claims.

## Claims

1. An intrabody fluid transfer system comprising:
a cannula assembly (10) comprising a proximal end (10p) with a connector (11) and having a longitudinally opposing distal end (10d) with an open channel extending therethrough; and
a surgical plunger assembly comprising:
a stylet (101) comprising opposing proximal and distal ends;
a connector (105) comprising an internal seal (103) adjacent the stylet; and
a support body (106) coupled to the connector (105) and extending above the distal end of the stylet,
wherein the support body (106) encloses a sub-length of the stylet; and
wherein the stylet (101) is longitudinally moveable relative to the support body (106) between first and second positions, wherein, in the first position, the proximal end of the stylet is closer to the connector (105) of the surgical plunger assembly than in the second position;
wherein the surgical plunger assembly (100) is configured to be coupled to the cannula assembly, wherein the stylet (101) extends in the open channel of the cannula assembly to position the distal end of the stylet adjacent the distal end of the cannula assembly, wherein the open channel and the stylet cooperate to define a fluid channel (10f) extending from the distal end (10d) of the cannula assembly (10); and
wherein the surgical plunger assembly (100) is configured to create a vacuum to intake a therapeutic substance at the distal end (10d) of the cannula assembly (10) in response to movement of the stylet (101) towards the second position to thereby provide a front-loaded therapeutic substance for delivery to a patient.

2. The intrabody fluid transfer system of Claim 1, further comprising a plunger comprising a first segment (102a) coupled to or defined by the stylet (101) and a second segment (102b) having a greater cross-sectional size that the first segment, wherein the second segment merges into an external plunger flange (110), wherein the support body (106) encloses the first segment and at least a portion of the second segment and the first segment and the second segment are longitudinally movable relative to the support body (106), and wherein with the plunger in a first position, the stylet (101) is in the first position and the second segment of the stylet resides closer to the connector (105) than when the stylet is in the second position.

3. The intrabody fluid transfer system of Claim 1, wherein the stylet (101) has a length outside the support body that is in a range of 152.4-3048 mm (6 inches to 10 feet).

4. The intrabody fluid transfer system of Claim 1 or Claim 2, wherein the stylet (101) comprises an MRI compatible material and has a maximal outer diameter in a range of about 0.127 to 0.508 mm (0.005 inches to about 0.020 inches),
optionally wherein the stylet (101) comprises Nitinol or wherein the stylet comprises fused silica.

5. The intrabody fluid transfer system of any preceding claim wherein the therapeutic substance comprises a drug for therapeutic treatment for Parkinson's disease.

6. The intrabody fluid transfer system of any preceding claim, further comprising a drive screw (220) that resides at least partially in the support body (106) and that is coupled to the proximal end of the stylet (101).

7. The intrabody fluid transfer system of Claim 6, further comprising a collar (210) that is coupled to the support body (106) and the drive screw (220), wherein the collar is rotatable in clockwise and counterclockwise directions to translate the stylet.

8. The intrabody fluid transfer system of Claim 2 or any claim dependent on Claim 2, further comprising a support tube (1101) residing inside the support body (106) and coupled to the connector above the seal (103), wherein the stylet is slidably coupled to the support tube to retract and extend inside the support tube while the support body slidably retracts and extends in concert with the stylet about an outer wall of the support tube.

9. The intrabody fluid transfer system according to any preceding claim, wherein the fluid channel (10f) extends from the distal end of the cannula assembly having a length in a range of about 1 cm to about 30 cm.

10. The intrabody fluid transfer system of any preceding claim, wherein, with the stylet in the first position associated with a ready to intake the therapeutic substance or a fully ejected position, the distal end of the stylet extends flush with or out of the distal end of the cannula assembly.

11. The intrabody fluid transfer system of Claim 10, wherein the therapeutic substance is held for dispensing to a patient entirely in the fluid channel at a location between the distal end of the cannula assembly and a medial portion of the cannula assembly.

12. The intrabody fluid transfer system of Claim 11, wherein the therapeutic substance comprises stem cells.

13. The intrabody fluid transfer system of any of Claims 1-11, wherein the therapeutic substance comprises target cells.

14. The intrabody fluid transfer system of any of the preceding claims, wherein the cannula assembly and the stylet are configured for deep brain placement for delivering the therapeutic substance to a target in a brain of a patient.

15. The intrabody fluid transfer system of any preceding claim, wherein a proximal end of the surgical plunger assembly is held above a trajectory guide of a surgical navigation system while a distal end of the surgical plunger assembly and the cannula assembly are below the proximal end of the surgical plunger assembly with the distal end of the cannula assembly and a distal end of the stylet configured to be in a brain of a patient for dispensing the therapeutic substance.

## Patentansprüche

1. An intrakorporales Fluidtransfersystem, das Folgendes beinhaltet:
eine Kanülenbaugruppe (10), die ein proximales Ende (10p) mit einem Anschluss (11) beinhaltet und ein längs gegenüberliegendes distales Ende (10d) mit einem sich dadurch erstreckenden offenen Kanal aufweist; und
eine chirurgische Kolbenbaugruppe, die Folgendes beinhaltet:
einen Mandrin (101), der einander gegenüberliegende proximale und distale Enden beinhaltet;
einen Anschluss (105), der eine Innendichtung (103) neben dem Mandrin beinhaltet; und
einen Stützkörper (106), der mit dem Anschluss (105) gekoppelt ist und sich über dem distalen Ende des Mandrins erstreckt,
wobei der Stützkörper (106) eine Teillänge des Mandrins umschließt; und wobei der Mandrin (101) relativ zu dem Stützkörper (106) zwischen einer ersten und einer zweiten Position längs beweglich ist, wobei das proximale Ende des Mandrins in der ersten Position näher an dem Anschluss (105) der chirurgischen Kolbenbaugruppe liegt als in der zweiten Position;
wobei die chirurgische Kolbenbaugruppe (100) dazu ausgelegt ist, mit der Kanülenbaugruppe gekoppelt zu sein, wobei sich der Mandrin (101) in dem offenen Kanal der Kanülenbaugruppe erstreckt, um das distale Ende des Mandrins neben dem distalen Ende der Kanülenbaugruppe zu positionieren, wobei der offene Kanal und der Mandrin kooperieren, um einen Fluidkanal (10f) zu definieren, der sich von dem distalen Ende (10d) der Kanülenbaugruppe (10) erstreckt; und
wobei die chirurgische Kolbenbaugruppe (100) dazu ausgelegt ist, ein Vakuum zum Aufnehmen einer therapeutischen Substanz an dem distalen Ende (10d) der Kanülenbaugruppe (10) als Reaktion auf eine Bewegung des Mandrins (101) hin zu der zweiten Position zu erzeugen, um somit eine von vorne geladene therapeutische Substanz zum Zuführen an einen Patienten bereitzustellen.

2. Intrakorporales Fluidtransfersystem gemäß Anspruch 1, das ferner einen Kolben beinhaltet, der ein erstes Segment (102a), das mit dem Mandrin (101) gekoppelt oder durch ihn definiert ist, und ein zweites Segment (102b), das eine größere Querschnittsgröße aufweist als das erste Segment, beinhaltet, wobei das zweite Segment in einen externen Kolbenflansch (110) übergeht, wobei der Stützkörper (106) das erste Segment und mindestens einen Abschnitt des zweiten Segments umschließt und das erste Segment und das zweite Segment relativ zu dem Stützkörper (106) längs beweglich sind, und wobei, wenn der Kolben in einer ersten Position ist, der Mandrin (101) in der ersten Position ist und sich das zweite Segment des Mandrins näher an dem Anschluss (105) befindet als wenn der Mandrin in der zweiten Position ist.

3. Intrakorporales Fluidtransfersystem gemäß Anspruch 1, wobei der Mandrin (101) eine Länge außerhalb des Stützkörpers aufweist, die im Bereich von 152,4-3048 mm (6 Zoll bis 10 Fuß) liegt.

4. Intrakorporales Fluidtransfersystem gemäß Anspruch 1 oder Anspruch 2, wobei der Mandrin (101) ein MRT-kompatibles Material beinhaltet und einen maximalen Außendurchmesser in einem Bereich von etwa 0,127 bis 0,508 mm (0,005 Zoll bis etwa 0,020 Zoll) aufweist,
wobei der Mandrin (101) optional Nitinol beinhaltet oder wobei der Mandrin Quarzglas beinhaltet.

5. Intrakorporales Fluidtransfersystem gemäß einem der vorherigen Ansprüche, wobei die therapeutische Substanz einen Wirkstoff zur therapeutischen Behandlung für die Parkinson-Krankheit beinhaltet.

6. Intrakorporales Fluidtransfersystem gemäß einem der vorherigen Ansprüche, das ferner eine Antriebsschraube (220) beinhaltet, die sich mindestens teilweise in dem Stützkörper (106) befindet und die mit dem proximalen Ende des Mandrins (101) gekoppelt ist.

7. Intrakorporales Fluidtransfersystem gemäß Anspruch 6, das ferner einen Bund (210) beinhaltet, der mit dem Stützkörper (106) und der Antriebsschraube (220) gekoppelt ist, wobei der Bund im Uhrzeigersinn und entgegen dem Uhrzeigersinn drehbar ist, um den Mandrin zu verschieben.

8. Intrakorporales Fluidtransfersystem gemäß Anspruch 2 oder einem von Anspruch 2 abhängigen Anspruch, das ferner ein Stützrohr (1101) beinhaltet, das sich innerhalb des Stützkörpers (106) befindet und mit dem Anschluss über der Dichtung (103) gekoppelt ist, wobei der Mandrin gleitfähig mit dem Stützrohr gekoppelt ist, um innerhalb des Stützrohrs zurückgezogen und ausgezogen zu werden, während der Stützkörper gleitfähig in Übereinstimmung mit dem Mandrin um eine Außenwand des Stützrohrs zurückgezogen und ausgezogen wird.

9. Intrakorporales Fluidtransfersystem gemäß einem der vorherigen Ansprüche, wobei sich der Fluidkanal (10f) von dem distalen Ende der Kanülenbaugruppe erstreckt, mit einer Länge in einem Bereich von etwa 1 cm bis etwa 30 cm.

10. Intrakorporales Fluidtransfersystem gemäß einem der vorherigen Ansprüche, wobei, wenn der Mandrin in der ersten Position ist, die mit einer für die Aufnahme der therapeutischen Substanz bereiten oder einer vollständig ausgeworfenen Position assoziiert ist, das distale Ende des Mandrins bündig abschließt mit oder sich aus dem distalen Ende der Kanülenbaugruppe heraus erstreckt.

11. Intrakorporales Fluidtransfersystem gemäß Anspruch 10, wobei die therapeutische Substanz zum Abgeben an einen Patienten gänzlich in dem Fluidkanal an einem Ort zwischen dem distalen Ende der Kanülenbaugruppe und einem mittleren Abschnitt der Kanülenbaugruppe gehalten wird.

12. Intrakorporales Fluidtransfersystem gemäß Anspruch 11, wobei die therapeutische Substanz Stammzellen beinhaltet.

13. Intrakorporales Fluidtransfersystem gemäß einem der Ansprüche 1-11, wobei die therapeutische Substanz Zielzellen beinhaltet.

14. Intrakorporales Fluidtransfersystem gemäß einem der vorherigen Ansprüche, wobei die Kanülenbaugruppe und der Mandrin zur Tiefenhirnplatzierung zum Zuführen der therapeutischen Substanz zu einem Ziel in einem Gehirn eines Patienten ausgelegt sind.

15. Intrakorporales Fluidtransfersystem gemäß einem der vorherigen Ansprüche, wobei ein proximales Ende der chirurgischen Kolbenbaugruppe über einer Bahnführung eines chirurgischen Navigationssystem gehalten wird, während ein distales Ende der chirurgischen Kolbenbaugruppe und die Kanülenbaugruppe unterhalb des proximalen Endes der chirurgischen Kolbenbaugruppe sind, wobei das distale Ende der Kanülenbaugruppe und ein distales Ende des Mandrins dazu ausgelegt sind, in einem Gehirn eines Patienten zu sein, um die therapeutische Substanz zuzuführen.

## Revendications

1. Système de transfert de fluide intracorporel comprenant :
un ensemble canule (10) comprenant une extrémité proximale (10p) avec un raccord (11) et ayant une extrémité distale (10d) opposée longitudinalement avec un canal ouvert s'étendant à travers elle ; et
un ensemble piston chirurgical comprenant :
un stylet (101) comprenant des extrémités proximale et distale opposées ;
un raccord (105) comprenant un joint (103) interne adjacent au stylet ; et
un corps de support (106) accouplé au raccord (105) et s'étendant au-dessus de l'extrémité distale du stylet,
dans lequel le corps de support (106) renferme une sous-longueur du stylet ; et dans lequel le stylet (101) est mobile longitudinalement par rapport au corps de support (106) entre des première et deuxième positions, dans lequel, dans la première position, l'extrémité proximale du stylet est plus près du raccord (105) de l'ensemble piston chirurgical que dans la deuxième position ;
dans lequel l'ensemble piston chirurgical (100) est configuré pour être accouplé à l'ensemble canule, dans lequel le stylet (101) s'étend dans le canal ouvert de l'ensemble canule pour positionner l'extrémité distale du stylet adjacente à l'extrémité distale de l'ensemble canule, dans lequel le canal ouvert et le stylet coopèrent pour définir un canal de fluide (10f) s'étendant à partir de l'extrémité distale (10d) de l'ensemble canule (10) ; et
dans lequel l'ensemble piston chirurgical (100) est configuré pour créer un vide afin d'admettre une substance thérapeutique à l'extrémité distale (10d) de l'ensemble canule (10) en réponse au déplacement du stylet (101) vers la deuxième position pour ainsi fournir une substance thérapeutique chargée par l'avant pour une administration à un patient.

2. Système de transfert de fluide intracorporel selon la revendication 1, comprenant en outre un piston comprenant un premier segment (102a) accouplé au stylet (101) ou défini par celui-ci et un deuxième segment (102b) ayant une taille en coupe transversale supérieure au premier segment, dans lequel le deuxième segment fusionne dans une bride de piston externe (110), dans lequel le corps de support (106) renferme le premier segment et au moins une portion du deuxième segment et le premier segment et le deuxième segment sont mobiles longitudinalement par rapport au corps de support (106), et dans lequel avec le piston dans une première position, le stylet (101) est dans la première position et le deuxième segment du stylet se trouve plus près du raccord (105) que lorsque le stylet est dans la deuxième position.

3. Système de transfert de fluide intracorporel selon la revendication 1, dans lequel le stylet (101) a une longueur à l'extérieur du corps de support qui est dans une plage de 152,4 à 3048 mm (6 pouces à 10 pieds).

4. Système de transfert de fluide intracorporel selon la revendication 1 ou la revendication 2, dans lequel le stylet (101) comprend un matériau compatible IRM et a un diamètre extérieur maximal dans une plage d'environ 0,127 à 0,508 mm (0,005 pouce à environ 0,020 pouce),
éventuellement dans lequel le stylet (101) comprend du nitinol ou dans lequel le stylet comprend du verre de silice.

5. Système de transfert de fluide intracorporel selon l'une quelconque des revendications précédentes dans lequel la substance thérapeutique comprend un médicament pour le traitement thérapeutique de la maladie de Parkinson.

6. Système de transfert de fluide intracorporel selon l'une quelconque des revendications précédentes, comprenant en outre un entraînement à vis (220) qui se trouve au moins partiellement dans le corps de support (106) et qui est accouplé à l'extrémité proximale du stylet (101).

7. Système de transfert de fluide intracorporel selon la revendication 6, comprenant en outre un collier (210) qui est accouplé au corps de support (106) et à l'entraînement à vis (220), dans lequel le collier est capable d'une rotation dans les sens horaire et antihoraire pour effectuer une translation du stylet.

8. Système de transfert de fluide intracorporel selon la revendication 2 ou l'une quelconque des revendications dépendantes de la revendication 2, comprenant en outre un tube de support (1101) se trouvant à l'intérieur du corps de support (106) et accouplé au raccord au-dessus du joint (103), dans lequel le stylet est accouplé coulissant au tube de support pour se rétracter et s'étendre à l'intérieur du tube de support tandis que le corps de support se rétracte et s'étend en coulissement de concert avec le stylet autour d'une paroi extérieure du tube de support.

9. Système de transfert de fluide intracorporel selon l'une quelconque des revendications précédentes, dans lequel le canal de fluide (10f) s'étend à partir de l'extrémité distale de l'ensemble canule ayant une longueur dans une plage d'environ 1 cm à environ 30 cm.

10. Système de transfert de fluide intracorporel selon l'une quelconque des revendications précédentes, dans lequel, avec le stylet dans la première position associée à une position prête à l'admission de la substance thérapeutique ou totalement éjectée, l'extrémité distale du stylet s'étend de niveau avec l'extrémité distale de l'ensemble canule ou hors de celle-ci.

11. Système de transfert de fluide intracorporel selon la revendication 10, dans lequel la substance thérapeutique est contenue pour une distribution à un patient entièrement dans le canal de fluide à un emplacement entre l'extrémité distale de l'ensemble canule et une portion médiane l'ensemble canule.

12. Système de transfert de fluide intracorporel selon la revendication 11, dans lequel la substance thérapeutique comprend des cellules souches.

13. Système de transfert de fluide intracorporel selon l'une quelconque des revendications 1 à 11, dans lequel la substance thérapeutique comprend des cellules cibles.

14. Système de transfert de fluide intracorporel selon l'une quelconque des revendications précédentes, dans lequel l'ensemble canule et le stylet sont configurés pour un placement en profondeur dans le cerveau afin d'administrer la substance thérapeutique à une cible dans le cerveau d'un patient.

15. Système de transfert de fluide intracorporel selon l'une quelconque des revendications précédentes, dans lequel une extrémité proximale de l'ensemble piston chirurgical est maintenue au-dessus d'un guide de trajectoire d'un système de navigation chirurgical tandis qu'une extrémité distale de l'ensemble piston chirurgical et l'ensemble canule sont au-dessous de l'extrémité proximale de l'ensemble piston chirurgical avec l'extrémité distale de l'ensemble canule et une extrémité distale du stylet configurées pour être dans le cerveau d'un patient afin de distribuer la substance thérapeutique.
